(19) Europäisches Patentamt
European Patent Office
Office européen des brevets

(11) Publication number: 0 189 941 B1

# (12) EUROPEAN PATENT SPECIFICATION

(45) Date of publication of patent specification: 26.09.90

(51) Int. Cl.[5]: A 61 K 31/545, A 61 K 47/00

(21) Application number: 86101308.4

(22) Date of filing: 31.01.86

## (54) Antibiotic compositions.

(30) Priority: 01.02.85 US 697362

(43) Date of publication of application:
06.08.86 Bulletin 86/32

(45) Publication of the grant of the patent:
26.09.90 Bulletin 90/39

(84) Designated Contracting States:
AT BE CH DE FR GB IT LI LU NL SE

(56) References cited:
EP-A-0 091 502
EP-A-0 101 170
DE-A-2 654 647
GB-A-2 072 675
US-A-4 237 279

RESEARCH DISCLOSURE, vol. 157, May 1977, abstract 15784, pages 75-76, Havant, UK; W.J. GOTTSTEIN et al.: "Certain cephalosporin salts"

(73) Proprietor: Bristol-Myers Squibb Company (a Delaware corp.)
345 Park Avenue
New York N.Y. 10154 (US)

(72) Inventor: Kaplan, Murray Arthur
1026 Glencove Road
Syracuse New York 13206 (US)
Inventor: Lipper, Robert Alan
7378 Barberry Lane
Manlius New York 13104 (US)

(74) Representative: Kinzebach, Werner, Dr.
Patentanwälte Reitstötter, Kinzebach und Partner Sternwartstrasse 4 Postfach 86 06 49
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 189 941 B1

Courier Press, Leamington Spa, England.

**Description**

This invention relates to a novel, stable, non-hygroscopic solvate of cefbuperazone, cefbuperazone hemiacetonate, and to pharmaceutical compositions comprising physical mixtures of cefbuperazone free acid or cefbuperazone hemiacetonate and at least one of lysine, lysine acetate, arginine and arginine acetate as defined below.

Cefbuperazone, a so-called "second generation" injectable cephalosporin, is disclosed in US—A—4,263,292 issued to I. Saikawa *et al.* on April 21, 1981 and has the following structure.

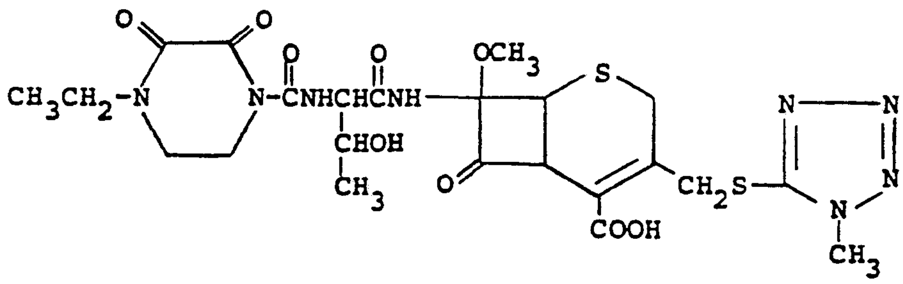

CEFBUPERAZONE

Patent 4,263,292 described the preparation of the dihydrate of cefbuperazone free acid (zwitterionic form), the lyophilized sodium salt of cefbuperazone and injectable pharmaceutical preparations containing the sodium salt.

European Patent Application EP—A—91,502, published October 19, 1983, discloses cephalosporin compositions for rectal administration which comprises one of a specific class of cephalosporins (including cefbuperazone), an ether type nonionic surfactant, an amino acid (including lysine and arginine) and an oleaginous base. The cephalosporins utilized are poorly absorbed when administered rectally. It is stated that, when administered in combination with an ether type nonionic surfactant, absorption is increased, but there is severe tissue damage. The addition of an amino acid to the composition reduces the rectal tissue damage. There is no suggestion of admixture of cefbuperazone and an amino acid without the surfactant and oleaginous base, no suggestion of an injectable form, and no discussion of pain on injection.

Although cefbuperazone is a useful antibiotic, the previously known forms have significant disadvantages. The free acid dihydrate is crystalline and stable in the dry state, but has a low solubility in water and therefore cannot be used to prepare aqueous injectable products having a reasonably useful cefbuperazone concentration. Also, the dihydrate is inconvenient to use for preparing pharmaceutical compositions because its water of hydration is very loosely bound, and its water content (and weight) varies with the ambient temperature and relative humidity. The lyophilized, amorphous sodium salt of cefbuperazone lacks sufficient stability for long-term storage, e.g. for periods in excess of one year, or for storage in areas of high ambient temperature. Moreover, injectable compositions of the sodium salt cause significant pain upon injection.

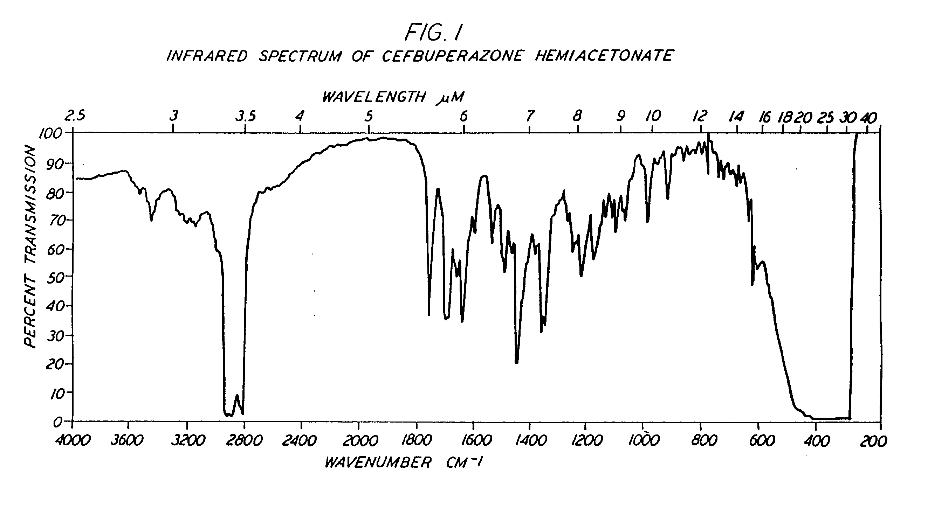

Figure 1 shows the infrared spectrum of cefbuperazone hemiacetonate as a mineral oil mull.

In their attempt to find a form of cefbuperazone which is stable in the dry state, water soluble and having reduced pain upon injection, applicants prepared and examined the sodium, potassium, calcium and ammonium salts of cefbuperazone, and salts prepared from cefbuperazone and the following bases: lidocaine, ethanolamine, triethanolamine, tris(hydroxymethyl)aminomethane, nicotinamide, ethylenediamine, N-methylglucamine, L(+)-lysine and L(+)-arginine. None of these yielded a crystalline product, and none of the amorphous products obtained offered any advantage, with respect to stability in the dry state, over the sodium salt of cefbuperazone.

Applicants then, surprisingly, discovered a solvate of cefbuperazone, the hemiacetonate, which is crystalline, non-hygroscopic and very stable in the dry state. Applicants also have unexpectedly found that, although the lysine and arginine salts of cefbuperazone are not stable in the dry state, physical mixtures of crystalline cefbuperazone dihydrate with lysine, lysine acetate, arginine or arginine acetate, or mixtures thereof, are stable in the dry state and water-soluble, and produce little or no pain upon injection. Cefbuperazone hemiacetonate is also advantageously used in physical admixture with lysine, lysine acetate, arginine or arginine acetate, or mixtures thereof, in view of its ability to retain a constant weight (i.e., not gain or lose water) under varying conditions of ambient temperature and relative humidity, and its low level of pain upon injection.

Table 1, below, shows the stability in the dry state of sodium cefbuperazone, cefbuperazone free acid, cefbuperazone hemiacetonate, and of mixtures of the latter two with lysine, arginine, lysine acetate or arginbine acetate.

Table 1

a) Lyophilized anhydrous sodium cefbuperazone

| Conditions | % Loss |
|---|---|
| 100°C - 1 day | melts/decomposes |
| 70°C - 3 days | 37.2 |
| 56°C - 2 weeks | 38.0 |
| 56°C - 4 weeks | 47.7 |
| 45°C - 4 weeks | 23.7 |
| 45°C - 8 weeks | 35.0 |

b) Cefbuperazone free acid dihydrate

| Conditions | % Loss |
|---|---|
| 100°C - 1 day | + 4.5 |
| 100°C - 2 days | + 3.3 |
| 56°C - 1 week | + 3.7 |
| 56°C - 2 weeks | + 4.6 |
| 56°C - 5 weeks | 0 |
| 56°C - 6 weeks | + 1.8 |
| 56°C - 8 weeks | + 2.2 |

c) Anhydrous cefbuperazone free acid

| Conditions | % Loss |
|---|---|
| 100°C - 1 day | 11 |
| 70°C - 1 day | 0 |
| 70°C - 3 days | 7.8 |
| 70°C - 1 week | 4.1 |
| 56°C - 4 weeks | 3.4 |

d) Cefbuperazone free acid hemiacetonate

| Conditions | % Loss |
|---|---|
| 100°C - 2 days | 0 |
| 70°C - 2 weeks | 0 |

e) Anhydrous cefbuperazone free acid (2 g) plus L(+) arginine acetate (0.968 g)

| Conditions | % Loss |
|---|---|
| 100°C - 1 day | 1.2 |
| 70°C - 3 days | 0.6 |
| 70°C - 1 week | 1.8 |
| 56°C - 1 month | 1.5 |

f) Cefbuperazone free acid dihydrate (0.5 g) plus L(+) lysine (0.135 g)

| Conditions | % Loss |
|---|---|
| 100°C - 1 day | 3.8 |
| 100°C - 2 days | 9 |
| 56°C - 2 weeks | 3.5 |
| 56°C - 5 weeks | 3.5 |
| 56°C - 6 weeks | 10 |
| 45°C - 5 weeks | 0 |
| 37°C - 5 weeks | 0 |

g) Cefbuperazone free acid hemiacetonate (1.023 g) plus L(+) arginine (0.27 g)

| Conditions | % Loss |
|---|---|
| 100°C - 1 day | 0 |
| 100°C - 2 days | 0 |
| 100°C - 3 days | 0 |
| 70°C - 3 days | 0 |
| 70°C - 1 week | 0 |
| 70°C - 2 weeks | 0 |
| 56°C - 2 months | 0 |

h) Cefbuperazone free acid hemiacetonate (0.565 g) plus L(+) lysine (0.131 g)

| Conditions | % Loss |
|---|---|
| 100°C - 1 day | 6.1 |
| 100°C - 2 days | 14.8 |
| 70°C - 3 days | 0.5 |
| 70°C - 1 week | 1.3 |
| 70°C - 2 weeks | 0 |
| 56°C - 8 weeks | 0 |
| 45°C - 8 weeks | 0 |

i) Cefbuperazone free acid hemiacetonate (2 g) plus L(+) lysine acetate (0.933 g)

| Conditions | % Loss |
|---|---|
| 70°C - 1 week | 7.6 |
| 56°C - 2 weeks | 5.2 |
| 56°C - 1 month | 1.2 |
| 56°C - 6 weeks | 1.5 |
| 56°C - 2 months | 3.3 |
| 37°C - 2 months | 0.6 |

Table 1 demonstrates the low stability of sodium cefbuperazone in the dry state. Cefbuperazone free acid dihydrate and anhydrous cefbuperazone free acid are significantly more stable, but are insufficiently water-soluble to be useful as a dry fill for reconstitution with water. Cefbuperazone hemiacetonate, and mixtures of cefbuperazone hemiacetonate, cefbuperazone free acid dihydrate or anhydrous cefbuperazone free acid with lysine, arginine, lysine acetate or arginine acetate, are shown to have good stability in the dry state, indicating excellent long-term shelf stability (e.g. greater than 1 year) under normal ambient conditions.

The amount of lysine, arginine, lysine acetate, arginine acetate, or mixtures thereof, to be mixed with cefbuperazone is not based on a molar equivalent basis, but is added in the amount necessary to produce a pH in the range of 4—7 when reconstituted with Sterile Water for Injection, U.S.P. The pH range preferably is in the range of 4—6 and most preferably is from 4 to 5.

It has been found that a pH of at least 3.5—3.7 upon reconstitution is necessary to insure complete solution of the dry mixture, it being preferable that the pH of the reconstituted mixture be at least about 4 to avoid the possibility of incomplete solution or possible crystallization in cool ambient conditions. The most preferred pH range of the reconstituted solution is 4 to 5, because pain upon injection normally increases with an increase of pH.

It will be appreciated that the purity (and hence, activity) of each batch of cefbuperazone will vary, whether dihydrate, anhydrous or hemiacetonate. Similarly, the purity of each batch of lysine, arginine, lysine acetate and arginine acetate will vary in purity (and hence, basicity). For this reason, it is not possible to give absolute gram amounts of cefbuperazone and lysine, arginine, lysine acetate and/or arginine acetate to produce a specific pH. The amount of each ingredient can readily be determined for individual batches of the two components by titration of the cefbuperazone with the other component. Table 2 shows typical results obtained when titrating cefbuperazone free acid dihydrate with L(+) lysine base. In this example, 264.4 mg of cefbuperazone free acid dihydrate (theoretically equal to 250 mg of cefbuperazone activity) was suspended in 10 ml of water and titrated with aqueous L(+) lysine. Solution of the cefbuperazone takes place at approximately pH 3.5.

Table 2

Titration of 264.4 mg of Cefbuperazone Free Acid Dihydrate

| pH | mg of L(+) lysine |
|---|---|
| 4.05 | 64.12 |
| 4.22 | 65.20 |
| 4.40 | 66.06 |
| 4.59 | 66.63 |
| 4.76 | 66.99 |
| 4.92 | 67.25 |
| 5.09 | 67.44 |
| 5.25 | 67.61 |
| 5.45 | 67.69 |
| 5.68 | 68.01 |
| 5.91 | 68.27 |
| 6.09 | 68.49 |
| 6.34 | 68.86 |

It will be seen that, with these particular batches of the two components, 264.4 mg of the cefbuperazone free acid dihydrate requires about 64.1 mg of L(+) lysine to give a reconstituted material having a pH of 4, about 66.0 mg to give a pH of 4.4, etc.

Table 3, below shows typical amounts of components, dilution volume (with Sterile Water for Injection, U.S.P.) and resulting pH of unit dosage forms containing 333 mg of cefbuperazone activity per ml.

Table 3

| Cefbuperazone | Amino Acid | Diluted to (ml) | pH of Solution |
|---|---|---|---|
| free acid · $2H_2O$ (1.023 g) | L(+) arginine (0.27 g) | 3.0 | 4.2 |
| free acid · $2H_2O$ (1.023 g) | L(+) lysine (0.238 g) | 3.0 | 5.0 |
| free acid · $2H_2O$ (1.13 g) | L(+) lysine (0.272 g) | 3.4 | 5.7 |
| free acid · $2H_2O$ (1.13 g) | L(+) arginine (0.312 g) | 3.4 | 5.9 |
| free acid · $2H_2O$ (1.0 g) | L(+) lysine acetate (0.467 g) | 3.4 | 4.2 |
| free acid · $2H_2O$ (1.0 g) | L(+) arginine acetate (0.48 g) | 3.4 | 4.2 |
| hemiacetonate (1.18 g) | L(+) lysine (0.24 g) | 3.0 | 4.2 |
| hemiacetonate (1.18 g) | L(+) arginine acetate (0.467 g) | 3.0 | 4.2 |
| sodium salt (1.102 g) | -- | 3.0 | 5.3 |

The novel, crystalline cefbuperazone hemiacetonate provided by this invention has been characterized by x-ray powder diffraction pattern, infrared spectrum, nuclear magnetic resonance spectrum and differential scanning calorimetry.

The nuclear magnetic resonance confirmed the presence of cefbuperazone and showed the presence of one-half mole of acetone per mole of cefbuperazone. This was shown to be bound acetone (a solvate) rather than free acetone by the fact that the acetone was retained even when heated at 50°C *in vacuo*. Under these same conditions, cefbuperazone dihydrate lost water of crystallization.

The x-ray powder diffraction pattern was determined on a Rigaku X-ray Powder Diffractometer using a Cu 25 kv lamp operated at 20 ma, at a scan rate of 2° per minute from 40° to 5° using a chart speed of 10 mm per minute and a nickel filter. The d-spacings and relative intensity of the peaks are shown in Table 4.

Table 4

X-ray Power Diffraction Pattern of Cefbuperazone Hemiacetonate

| d-Spacing (Å) | Relative Intensity ($I/I_o$ %) |
|---|---|
| 9.50 | 81 |
| 9.30 | 100 |
| 7.62 | 13 |
| 6.65 | 21 |
| 6.23 | 17 |
| 5.75 | 35 |
| 5.40 | 17 |
| 5.03 | 21 |
| 4.92 | 25 |
| 4.71 | 15 |
| 4.54 | 27 |
| 4.23 | 60 |
| 3.86 | 33 |
| 3.53 | 19 |
| 3.25 | 21 |

The infrared absorption spectrum of cefbuperazone hemiacetonate (as a mineral oil mull) is shown in Figure 1 hereof.

The differential scanning calorimeter curve of cefbuperazone showed endotherms at 163°C and 180°C, and an exotherm at 185°C.

The process for preparing cefbuperazone free acid hemiacetonate comprises

a) adjusting the pH of a slurry of cefbuperazone free acid in water to 3—6

b) adding acetone to the solution,

c) adjusting the pH to 1—3 and

d) isolating the product.

Preparation No. 1

Preparation of Sterile Cefbuperazone Free Acid (Anhydrous Form)

1. Slurry 10 g of hydrated cefbuperazone free acid in 25 ml of Water for Injection, U.S.P. (22—26°C).

2. With very rapid stirring, slowly add a 7.5% w/v solution of $NaHCO_3$ until a solution or near solution and a pH of 4.0—5.0 are obtained. Approximately 15 ml is required.

3. Add 10 ml of absolute ethanol. Stir for 5 minutes.

4. Using aseptic technique, pass the solution through an appropriate sterile, 0.22 (μm) pore size membrane filter and into an appropriate sterile container.

5. With very rapid stirring and over at least a 10-minute interval, add sterile, 6—12N HCl to a pH of 2.5—3.0. Crystals form. Stir for 0.5 hours.

6. Vigorously stir the mixture for 1—2 hours at a maintained pH of 1.5—2.5 by addition of 6—12N HCl, if required.

7. Vacuum filter the crystals through an appropriate sterile filtration unit.

8. Tamp the crystals to smooth the filter cake and remove any cracks which may have formed.

9. Wash the crystals with two × 10 ml portions of 75% Sterile Water for Injection, U.S.P./25% ethanol, v/v. Assure that any cracks present in the filter cake are removed by tamping before each washing.

10. Remove all free liquid from the filter cake by tamping smooth under vacuum.

11. Wash the filter cake with three × 10 ml portions of sterile ethanol. Tamp the filter cake smooth after each washing.

12. Maintain vacuum until all free ethanol appears removed.

13. Vacuum dry the crystals aseptically at 55—65°C for 24 hours. Expected yield: 9.0—9.5 g of sterile cefbuperazone free-acid, anhydrous form.

Preparation No. 2

Preparation of Sterile Cefbuperazone Free Acid Dihydrate

1. Repeat Steps 1—8 of Preparation No. 1.

2. Wash the resulting crystals with two × 10 ml portions of Sterile Water for Injection, U.S.P. Assure that any cracks present in the filter cake are removed by tamping before each washing.

3. Remove all free liquid from the filter cake by tamping smooth under vacuum.

4. Air dry the crystals aseptically at 20—25°C and relative humidity >50% for 24 hours. Expected yield 9.5—9.7 g.

Preparation No. 3

Preparation of Sterile, Crystalline L-Arginine Free Base

1. Dissolve 3 g of L-arginine free base in 30 ml of Water for Injection, U.S.P.

2. Using aseptic technique, pass the solution through a suitable sterile 0.22 μm (micron) pore size filter.

3. Add the filtered solution, over a 10-minute interval, to 400 ml of rapidly stirring, sterile isopropanol. Crystals form. Stir for 1 hour.

4. Isolate the crystals via a suitable aseptic vacuum filtration procedure.

5. Wash the crystals with 80 ml of sterile isopropanol.

6. Vacuum-dry the crystals under aseptic conditions at 65—70°C for 24 hours. Expected yield 2.7 g. Store in a suitable sterile container.

Preparation No. 4

Preparation of Sterile, Crystalline L-Arginine Acetate

1. Slurry 10 g of L-arginine free base in 30 ml of Water for Injection, U.S.P.

2. With rapid stirring, add 12.2 ml (approximately 3 molar equivalents) of glacial acetic acid over a 5-minute interval. A solution or near solution is obtained. Cool to 20—25°C.

3. Using aseptic technique, pass the solution through a suitable sterile 0.22 μm (micron) pore size filter.

4. Add the filtered solution, over a 15-minute interval, to 500 ml of rapidly stirring, sterile absolute ethanol. Crystals form. Stir for 0.5 hour.

5. Isolate the crystals via a suitable aseptic vacuum filtration procedure.

6. Wash the crystals with 100 ml of sterile absolute ethanol.

7. Vacuum-dry the crystals under aseptic conditions at 65—75°C for 24 hours. Expected yield: 10.1 g. Store in a suitable sterile container.

Preparation No. 5

Preparation of Sterile, Crystalline L-Lysine

1. Slurry 50 grams of L-lysine base in 500 ml of methanol for 20 minutes at 20—25°C.

2. Remove the insolubles (mostly L-lysine carbonate) by suitable filtration technique. At this point, the filtrate may have a slight haze. Discard the filter cake.

3. With rapid stirring, add 7.5 grams of decolorizing carbon to the filtrate of Step 2 and stir for 0.5 hour.

4. Remove the carbon by suitable filtration technique.

5. Wash the filter cake with 75 ml of methanol. Add the wash to the filtrate.

6. Pass the filtrate-wash through a suitable 0.22 μm (micron) Millipore® filter to remove bacteria and particles. Collect the filtrate in suitable sterile, pyrogen-free containers (glass or stainless steel).

7. In a sterile environment, using sterile techniques, add, with very rapid stirring (air stirrer), 2 liters of sterile, pyrogen-free isopropanol over a one-hour interval to the sterile, pyrogen-free methanol solution of L-lysine base of Step 6. A heavy precipitate forms. Slurry an additional 15 minutes.

8. Remove the crystalline L-lysine base by suitable, sterile filtration techniques. Tamp the filter cake to remove cracks and fissures.

9. Wash the filter cake with 200 ml of sterile, pyrogen-free isopropanol and continue the vacuum until the cake appears dry.

10. High-vacuum dry the filter cake at 56°—65°C for 24 hours. Yield: approximately 40 grams.

11. Mill or screen the solids by sterile techniques to 40—60 mesh.

12. Re-dry the solids at high-vacuum, 56°—65°C for 24 hours.

13. Store the solids in sterile, pyrogen-free amber bottles.

14. If desired or required, the isopropanol used in Steps 7 and 9 may be substituted with equal volumes of sterile, pyrogen-free ethyl acetate.

Properties of Parenteral Grade L-Lysine Base

1. IR—NMR: a. Consistent for structure
b. Substantially free of methanol, ethyl acetate, isopropanol

2. % Water, Karl Fischer: 1—2%

Preparation No. 6

Preparation of Sterile, Crystalline L-Lysine Acetate

1. Dissolve 10 g of L-lysine free base in 30 ml of Water for Injection, U.S.P.

2. With rapid stirring, add 12.2 ml (3 molar equivalents) of glacial acetic acid over a 5-minute interval. Cool to 20—25°C.

3. Using aseptic technique, pass the solution through a suitable sterile 0.22 μm (micron) pore size filter.

4. Add the filtered solution, over a 15-minute interval, to 500 ml of rapidly stirring, sterile isopropanol. Crystals form. Stir for 0.5 hour.

5. Isolate the crystals via a suitable aseptic vacuum filtration procedure.

6. Wash the crystals with 75 ml of sterile isopropanol.

7. Vacuum-dry the crystals under aseptic conditions at 65—75°C for 24 hours. Expected yield: 9.6 g. Store in a suitable sterile container.

Example 1

Dry Fill Mixture: Cefbuperazone Free Acid (Anhydrous) + L-Arginine Free Base for Parenteral Use (Label claim per vial is 250 mg of cefbuperazone activity)

All work should be conducted under aseptic conditions at a relative humidity of below 50%, preferably at 30—35%

Formula Per Vial

| Compound | Weight |
|---|---|
| 1. Sterile, 60—100 mesh, cefbuperazone free acid (anhydrous) | *250 mg (250 mg of cefbuperazone activity) |
| 2. Sterile, 60—100 mesh, anhydrous, L-arginine free base | **63—70 mg |

*This weight assumes a 100% purity. To determine the amount of cefbuperazone free acid required, use the following formula:

$$\frac{1000}{\text{Potency of cefbuperazone free acid (anhydrous) in } \mu g/mg} \times 250 = \text{mg of cefbuperazone free acid required.}$$

**This amount of L-arginine free base is required to give a reconstituted pH of 4.0—7.0 (pH 4.0—6.0 preferred) in Sterile Water for Injection, U.S.P. The actual amount of L-arginine free base required can vary as a function of its purity, desired pH and the potency of the cefbuperazone free acid.

Manufacturing Instructions

1. The aseptic manufacturing area relative humidity must be less than 50%, preferably 30—35%.
2. Using aseptic technique, suitably blend the batch-weight requirements of the sterile cefbuperazone free acid (anhydrous) and L-arginine free base.
3. Drop the blend in a suitable sterile holding container. Submit the potency, sterility, blend uniformity and testing for other required parameters.
4. Aseptically fill the required amount*** of bulk-blend into appropriate vials. Seal with appropriate rubber closures and aluminum shells.

***The actual weight of fill can vary as a function of needle-vial-syringe retention and additional overfill required.

Example 2

Dry Fill Mixture: Cefbuperazone Free Acid·$2H_2O$ + L(+) Lysine Base for Parenteral Use

(Label claim per vial is 250 mg of cefbuperazone activity)

All work should be conducted under aseptic conditions at a relative humidity of below 50%, preferably at 30—35%.

Formula Per Vial

| Compound | Weight |
|---|---|
| 1. Sterile, 60—100 mesh, cefbuperazone·$2H_2O$ free acid | *264.34 mg (250 mg of cefbuperazone activity) |
| 2. Sterile, 60—100 mesh, anhydrous, L(+) lysine free base | **65—70 mg |

*This weight assumes a 100% purity. To determine the amount of cefbuperazone·$2H_2O$ free acid required, use the following formula:

$$\frac{946}{\text{Potency of cefbuperazone}\cdot 2H_2O\text{ free acid in }\mu g/mg} \times 264.34 = \text{mg of cefbuperazone}\cdot 2H_2O\text{ free acid required}$$

**This amount of L(+) lysine base is required to give a reconstituted pH of 4.0—6.0 (pH 4.6—5.4 preferred) in Sterile Water for Injection, U.S.P., qs to 1.0 ml. The actual amount of L(+) lysine free base required can vary as a function of its purity, desired pH and the potency of the cefbuperazone·$2H_2O$ free acid.

Manufacturing Instructions

1. The aseptic manufacturing area relative humidity must be less than 50%, preferably 30—35%.
2. Using aseptic technique, suitably blend the batch-weight requirements of the sterile, cefbuperazone·$2H_2O$ and L(+) lysine free base.
3. Drop the blend in a suitable sterile holding container. Submit for potency, sterility, blend uniformity and testing for other required parameters.
4. Aseptically fill the required amount*** of bulk-blend into appropriate vials. Seal with appropriate rubber closures and aluminum shells.

*** The actual weight of fill can vary as a function of needle-vial-syringe retention and additional overfill required.

Example 3

Dry Fill Mixture: Cefbuperazone Free Acid (Anhydrous) + L(+) Lysine Free Base for Parenteral Use

(Label claim per vial is 250 mg of cefbuperazone activity)

All work should be conducted under aseptic conditions at a relative humidity of below 50%, preferably at 30—35%.

Formula Per Vial

| Compound | Weight |
|---|---|
| 1. Sterile, 60—100 mesh, cefbuperazone free acid (anhydrous) | *250 mg (250 mg of cefbuperazone activity) |
| 2. Sterile, 60—100 mesh, anhydrous, L(+) lysine free base | **54—61 mg |

*This weight assumes a 100% purity. To determine the amount of cefbuperazone free acid required, use the following formula:

$$\frac{1000}{\text{Potency of cefbuperazone free acid in } \mu g/mg} \times 250 = \text{mg of cefbuperazone free acid required}$$

**This amount of L(+) lysine base is required to give a reconstituted pH of 4.0—6.0 (pH 4.0—5.5 preferred) in Sterile Water for Injection, U.S.P. The actual amount of L(+) lysine free base required can vary as a function of its purity, desired pH and the potency of the cefbuperazone free acid.

Manufacturing Instructions

1. The aseptic manufacturing area relative humidity must be less than 50%, preferably 30—35%.
2. Using aseptic technique, suitably blend the batch-weight requirements of the sterile, cefbuperazone free acid (anhydrous) and L(+) lysine free base.
3. Drop the blend in a suitable sterile holding container. Submit for potency, sterility, blend uniformity and testing for other required parameters.
4. Aseptically fill the required amount*** of bulk-blend into appropriate vials. Seal with appropriate rubber closures and aluminum shells.

---

*** The actual weight of fill can vary as a function of needle-vial-syringe retention and additional overfill required.

### Example 4

Preparation of Sterile Cefbuperazone Hemi-Acetonate

1. Slurry 10 g of hydrated cefbuperazone free acid in 25 ml of Water for Injection, U.S.P. (22—26°C).
2. With very rapid stirring, slowly add a 7.5% w/v solution of $NaHCO_3$ until a solution or near solution and a pH of 4.0—5.0 are obtained. Approximately 15 ml is required.
3. Add 10 ml of acetone. Stir for 5 minutes.
4. Using aseptic technique, pass the solution through an appropriate, sterile 0.22 µm (micron) pore size membrane filter and into an appropriate sterile container.
5. With very rapid stirring and over at least a 10-minute interval, add sterile, 6—12 N HCl to a pH of 2.5—3.0. Crystals form. Stir for 0.5 hour.
6. Vigorously stir the mixture for 1—2 hours at a maintained pH of 1.5—2.5 by addition of 6—12 N HCl, if required.
7. Vacuum filter the crystals through an appropriate sterile filtration unit.
8. Tamp the crystals to smooth the filter cake and remove any cracks which may have formed.
9. Wash the crystals with 2 × 10 ml portions of 75% Sterile Water for Injection, U.S.P./25% sterile acetone, v/v. Assure that any cracks present in the filter cake are removed by tamping before each washing.
10. Remove all free liquid from the filter cake by tamping smooth under vacuum.
11. Wash the filter cake with 2 × 10 ml portions of sterile acetone. Tamp the filter cake smooth after each washing.
12. Slurry the acetone-damp crystals in 30—40 ml of acetone for 0.5 hour. Remove the crystals by vacuum filtration. Maintain vacuum until all free acetone appears removed.
13. Vacuum dry the crystals aseptically at 40—50°C for 24 hours. Expected yield: 9.0—9.5 g of sterile cefbuperazone hemiacetonate.

In preparing the compositions of this invention, one may use racemic lysine or arginine (or their acetate) or their D(−) or L(+) isomers. Since the L(+) isomers are the commonly available, naturally occurring forms, we prefer to use the L(+) isomers. Preferably, the lysine or arginine (or their acetate) is substantially dry, i.e. containing less than 1—2% water. In order to minimize the volume of solution to be injected, we prefer to reconstitute the compositions to a volume of 300 to 350 mg of cefbuperazone activity per ml of solution. The compositions may be reconstituted with Sterile Water for Injection, U.S.P., or other common injection vehicles such as 5% glucose or normal saline.

The dosage of the compositions of this invention depend on the age, weight and general health of the patient, as well as the severity of the disease, and is within the discretion of the physician. Generally, however, the dosages are similar to other known injectable cephalosporins, e.g. from 250 mg to 1 g, administered every six hours. In very serious or life-threatening infections, up to 10—12 grams per day may be administered.

Intravenous toxicity tests were conducted in Swiss-Webster mice using the sodium salt of cefbuperazone and the compositions of the present invention. The Lethal $Dose_{50}$ ($LD_{50}$) for each of the tested compositions is shown in Table 5. In each case, the dosing solution contained 333 mg of cefbuperazone activity per ml.

Table 5

Intravenous Toxicity (Mice)

| Test Material | $LD_{50}$ (mg/kg) |
|---|---|
| sodium cefbuperazone | >5000 |
| cefbuperazone free acid · $2H_2O$ (1.13 g)<br>L(+) lysine (0.246 g) | >5000 |
| cefbuperazone free acid · $2H_2O$ (1.13 g)<br>L(+) arginine (0.285 g) | >5000 |
| cefbuperazone free acid · $2H_2O$ (1.13 g)<br>L(+) lysine acetate (0.467 g) | 1857 (1595-2567) |
| cefbuperazone free acid · $2H_2O$ (1.13 g)<br>L(+) arginine acetate (0.48 g) | 2136 (1796-2436) |

Note: Numbers in parentheses are 95% confidence limits

The compositions of this invention were assessed for pain on injection by a modification of the rat-paw lick test described by E. Gelozzi *et al.,* Journal of Pharmacological Methods, *4,* 285—9 (1980). The test animals were weanling female Sprague-Dawley rats. The test solutions were injected into the subplantar tissue of the right or left hind paw, using a syring with a 27 gauge, 1.27 cm (½ inch) needle. The injection volume in each case was 0.1 ml, using a solution reconstituted with Sterile Water for Injection, U.S.P. to a concentration of 333 mg of cefbuperazone activity per ml. The paw-lick response, an indicator of the pain caused by the injection, was recorded for each rat for three minutes following the injection. A paw-licking

episode is defined as a licking response followed by a turning of the head away from the paw or five seconds of uninterrupted licking. The results of the tests are shown in Table 6.

Table 6

Rat Paw-Lick Test for Pain on Injection

| Test Material | pH | Paw-Lick Response[1] |
|---|---|---|
| Sterile Water for Injection, U.S.P. | | 1.48 ± 0.18 (110) |
| sodium cefbuperazone | 5.3 | 5.20 ± 1.31 (10) |
| cefbuperazone hemiacetonate + L-lysine | 4.2 | 1.83 ± 0.50 (30) |
| cefbuperazone free acid · $2H_2O$ + L-lysine | 5.7 | 4.00 ± 1.10 (10) |
| cefbuperazone free acid · $2H_2O$ + L-lysine | 7.5 | 7.40 ± 1.20 (10) |
| cefbuperazone free acid · $2H_2O$ + L-lysine acetate | 4.2 | 4.40 ± 1.13 (10) |
| cefbuperazone hemiacetonate + L-arginine | 4.2 | 0.80 ± 0.32 (20) |
| cefbuperazone free acid · $2H_2O$ + L-arginine | 5.9 | 2.10 ± 0.66 (10) |
| cefbuperazone free acid · $2H_2O$ + L-arginine | 7.5 | 3.20 ± 1.23 (10) |
| cefbuperazone free acid · $2H_2O$ + L-arginine acetate | 4.2 | 5.10 ± 0.95 (10) |

[1]Mean paw-lick response 0-3 minutes after injection ± Standard Error. Numbers in parentheses are the number of test animals.

It should be noted that, at a given pH of the reconstituted solution, no difference in pain level would be expected between compositions made from dry mixes containing cefbuperazone free acid dihydrate, anhydrous cefbuperazone free acid or cefbuperazone free acid hemiacetonate. This is because the first two are identical in aqueous solution, while the third differs only in that the aqueous solution will contain a trace of acetone.

**Claims for the Contracting States: BE CH DE FR GB IT LI LU NL SE**

1. A stable, sterile cefbuperazone dry mix composition for reconstitution with a sterile, pharmaceutically acceptable diluent to produce an injectable solution having low pain upon administration, which comprises:

a) a therapeutically effective amount of cefbuperazone free acid dihydrate, anhydrous cefbuperazone free acid or cefbuperazone free acid hemiacetonate, and

b) lysine, arginine, lysine acetate, arginine acetate, or a mixture thereof, in an amount sufficient to produce a reconstituted solution having a pH of from 4 to 7.

2. A composition of Claim 1 in which the lysine, arginine, lysine acetate, arginine acetate, or mixture thereof, is present in an amount sufficient to produce a reconstituted solution having a pH of from 4 to 6.

3. A stable, sterile cefbuperazone dry mix composition for reconstitution with a sterile, pharmaceutically acceptable diluent to produce an injectable solution having low pain upon administration, which comprises:

a) a therapeutically effective amount of cefbuperazone free acid dihydrate, anhydrous cefbuperazone free acid or cefbuperazone free acid hemiacetonate, and

b) lysine or arginine in an amount sufficient to produce a reconstituted solution having a pH of from 4 to 5.

4. A composition of claim 1 in which the lysine or arginine in an amount sufficient to produce a reconstituted aqueous solution having a pH of from 4 to 5.

5. A compositon of Claim 4 in unitary dosage form containing, when reconstituted, from about 300 to about 350 mg of cefbuperazone activity per ml of reconstituted solution.

6. Cefbuperazone free acid hemiacetonate.

7. A process for preparing cefbuperazone free acid hemiacetonate of claim 6, which comprises

a) adjusting the pH of a slurry of cefbuperazone free acid in water to 3—6

b) adding acetone to the solution,

c) adjusting the pH to 1—3 and

d) isolating the product.

8. A process for preparing the compositions of claims 1—5, which comprises mixing the components a) and b) in an amount sufficient to produce a reconstituted solution having the desired pH of at least 3.5—3.7.

**Claims for the Contracting State: AT**

1. A process for preparing a stable, sterile cefbuperazone dry mix composition for reconstitution with a sterile, pharmaceutically acceptable diluent to produce an injectable solution having low pain upon administration, which comprises mixing

a) a therapeutically effective amount of cefbuperazone free acid dihydrate, anhydrous cefbuperazone free acid or cefbuperazone free acid hemiacetonate, and

b) lysine, arginine, lysine acetate, arginine acetate, or a mixture thereof, in an amount sufficient to produce a reconstituted solution having a pH of from 4 to 7.

2. The process of Claim 1 in which the lysine, arginine, lysine acetate, arginine acetate, or mixture thereof, is present in an amount sufficient to produce a reconstituted solution having a pH of from 4 to 6.

3. The process for preparing a stable, sterile cefbuperazone dry mix composition for reconstitution with a sterile, pharmaceutically acceptable diluent to produce an injectable solution having low pain upon administration, which comprises mixing

a) a therapeutically effective amount of cefbuperazone free acid dihydrate, anhydrous cefbuperazone free acid or cefbuperazone free acid hemiacetonate, and

b) lysine or arginine in an amount sufficient to produce a reconstituted solution having a pH of from about 4 to about 5.

4. The process of claim 1 in which lysine or arginine are present in an amount sufficient to produce a reconstituted aqueous solution having a pH of from 4 to 5.

5. The process of Claim 4 for preparing a composition in unitary dosage form containing, when reconstituted, from about 300 to about 350 mg of cefbuperazone activity per ml of reconstituted solution.

6. A process for preparing cefbuperazone free acid hemiacetonate which comprises

a) adjusting the pH of a slurry of cefbuperazone free acid in water to 3—6

b) adding acetone to the solution,

c) adjusting the pH to 1—3 and

d) isolating the product.

7. The process of claims 1 to 5 which comprises mixing the components a) and b) in an amount sufficient to produce a reconstituted solution having the desired pH of at least 3.5 to 3.7.

**Patentansprüche für die Vertragsstaaten: BE DE FR IT LU NL SE CH LI GB**

1. Stabile sterile cefbuperazonhaltige Trockenmischung zur Rekonstitution mit einem sterilen, pharmazeutisch verträglichen Verdünnungsmittel zur Bildung einer injizierbaren Lösung, bei deren Verabreichung der Schmerz gering ist, umfassend:

a) eine therapeutisch wirksame Menge des Dihydrats der freien Säure von Cefbuperazon, wasserfreies Cefbuperazon in Form der freien Säure oder des Hemiacetonats der freien Säure von Cefbuperazon, und

b) Lysin, Arginin, Lysinacetat, Argininacetat oder eine Mischung davon, in einer Menge, die zur Bildung einer rekonstituierten Lösung mit einem pH-Wert von 4 bis 7 ausreicht.

2. Zusammensetzung nach Anspruch 1, worin Lysin, Arginin, Lysinacetat, Argininacetat oder eine Mischung davon in einer Menge vorliegen, die zur Bildung einer rekonstituierten Lösung mit einem pH-Wert von 4 bis 6 ausreicht.

3. Stabile, sterile cefbuperazonhaltige Trockenmischung zur Rekonstitution mit einem sterilen, pharmazeutisch verträglichen Verdünnungsmittel zur Bildung einer injizierbaren Lösung, bei deren Verabreichung der Schmerz gering ist, umfassend:

a) eine therapeutisch wirksame Menge des Dihydrats der freien Säure von Cefbuperazon, wasserfreies Cefbuperazon in Form der freien Säure oder des Hemiacetonats der freien Säure von Cefbuperazon, und

b) Lysin oder Arginin in einer Menge, die zur Bildung einer rekonstituierten Lösung mit einem pH-Wert von 4 bis 5 ausreicht.

4. Zusammensetzung nach Anspruch 1, worin Lysin oder Arginin in einer Menge vorliegen, die zur Bildung einer rekonstituierten wässrigen Lösung mit einem pH-Wert von 4 bis 5 ausreicht.

5. Zusammensetzung nach Anspruch 4 in Einheitsdosisform, die, nach Rekonstitution, etwa 300 bis etwa 350 mg Cefbuperazon-Aktivität pro ml rekonstituierte Lösung aufweist.

6. Hemiacetonat der freien Säure von Cefbuperazon.

7. Verfahren zur Herstellung des Hemiacetonats der freien Säure von Cefbuperazon nach Anspruch 6, wobei

a) der pH-Wert einer Aufschlämmung der freien Säure von Cefbuperazone in Wasser auf 3 bis 6 eingestellt wird,

b) Aceton der Lösung zugesetzt wird,

c) der pH-Wert auf 1 bis 3 eingestellt wird und

d) das Produkt isoliert wird.

8. Verfahren zur Herstellung der Zusammensetzungen nach den Ansprüchen 1 bis 5, wobei die Komponenten a) und b) in einer Menge gemischt werden, die zur Bildung einer rekonstituierten Lösung mit dem gewünschten pH-Wert von mindestens 3.5 bis 3.7 ausreicht.

**Patentansprüche für den Vertragsstaat AT**

1. Verfahren zur Herstellung einer stabilen, sterilen cefbuperazonhaltigen Trockenmischung zur Rekonstitution mit einem sterilen, pharmazeutisch verträglichen Verdünnungsmittel zur Bildung einer injizierbaren Lösung, bei deren Verabreichung der Schmerz gering ist, wobei man

a) eine therapeutisch wirksame Menge des Dihydrats der freien Säure von Cefbuperazon, wasserfreies Cefbuperazon in Form der freien Säure oder des Hemiacetonats der freien Säure von Cefbuperazon, und

b) Lysin, Arginin, Lysinacetat, Argininacetat oder eine Mischung davon, in einer Menge, die zur Bildung einer rekonstituierten Lösung mit einem pH-Wert von 4 bis 7 ausreicht.

2. Verfahren nach Anspruch 1, worin Lysin, Arginin, Lysinacetat, Argininacetat oder eine Mischung davon in einer Menge vorliegen, die zur Bildung einer rekonstituierten Lösung mit einem pH-Wert von 4 bis 6 ausreicht.

3. Verfahren zur Herstellung einer stabilen, sterilen cefbuperazonhaltigen Trockenmischung zur Rekonstitution mit einem sterilen, pharmazeutisch verträglichen Verdünnungsmittel zur Bildung einer injizierbaren Lösung, bei deren Verabreichung der Schmerz gering ist, wobei man

a) eine therapeutisch wirksame Menge des Dihydrats der freien Säure von Cefbuperazon, wasserfreies Cefbuperazon in Form der freien Säure oder des Hemiacetonats der freien Säure von Cefbuperazon, und

b) Lysin oder Arginin in einer Menge vermischt, die zur Bildung einer rekonstituierten Lösung mit einem pH-Wert von 4 bis 5 ausreicht.

4. Verfahren nach Anspruch 1, worin Lysin oder Arginin in einer Menge vorliegen, die zur Bildung einer rekonstituierten wässrigen Lösung mit einem pH-Wert von 4 bis 5 ausreicht.

5. Verfahren nach Anspruch 4 zur Herstellung einer Zusammensetzung in Einheitsdosisform, die, nach Rekonstitution, etwa 300 bis etwa 350 mg Cefbuperazon-Aktivität pro ml rekonstituierte Lösung aufweist.

6. Verfahren zur Herstellung des Hemiacetonats der freien Säure von Cefbuperazon, wobei

a) der pH-Wert einer Aufschlämmung der freien Säure von Cefbuperazone in Wasser auf 3 bis 6 eingestellt wird,

b) Aceton der Lösung zugesetzt wird,

c) der pH-Wert auf 1 bis 3 eingestellt wird und

d) das Produkt isoliert wird.

7. Verfahren nach den Ansprüchen 1 bis 5, wobei die Komponenten a) und b) in einer Menge gemischt werden, die ausreicht, um eine rekonstituierte Lösung mit dem gewünschten pH-Wert von mindestens 3.5 bis 3.7 zu erhalten.

**Revendications pour les Etats contractants: BE CH DE FR GB IT LI LU NL SE**

1. Composition de mélange de cefbupérazone, stérile et stable, pour reconstitution avec un diluant stérile et pharmaceutiquement acceptable, pour produire une solution injectable, dont l'administration est peu douloureuse, qui comprend

a) une quantité thérapeutiquement active du dihydrate de l'acide libre de cefbupérazone, de l'acide libre de cefbupérazone anhydre, ou du hemi-acétonate de l'acide libre de cefbupérazone, et

b) de la lysine, de l'arginine, de l'acétate de lysine, de l'acétate d'arginine, ou un de leurs mélanges, en une quantité suffisante pour produire une solution reconstituée ayant un pH de 4 à 7.

2. Composition selon la revendication 1, dans laquelle la lysine, l'arginine, l'acétate de lysine, l'acétate d'arginine, ou un de leurs mélanges, est présent en une quantité suffisante pour produire une solution reconstituée ayant un pH de 4 à 6.

3. Composition de mélange sec de cefbupérazone, stérile et stable, pour reconstitution avec un diluant stérile et pharmaceutiquement acceptable, pour produire une solution injectable, dont l'administration est

peu douloureuse, qui comprend

a) une quantité thérapeutiquement active du dihydrate de l'acide libre de cefbupérazone, de l'acide libre de cefbupérazone anhydre, ou du hemi-acétonate de l'acide libre de cefbupérazone, et

b) de la lysine ou de l'arginine, en une quantité suffisante pour produire une solution reconstituée ayant un pH de 4 à 5.

4. Composition selon la revendication 1, dans lequelle la lysine ou l'arginine sont présentes en une quantité suffisante pour produire une solution aqueuse reconstituée ayant un pH 4 à 5.

5. Composition selon la revendication 4, en doses individuelles, contenant, lorsqu'elle est reconstituée, d'environ 300 à environ 350 mg d'activité de cefbupérazone par ml de solution reconstituée.

6. Hemi-acétonate de l'acide libre de cefbupérazone.

7. Procédé pour la preparation du hemi-acétonate de l'acide libre de cefbupérazone, selon la revendication 6, comprenant les opérations consistant à:

a) ajuster le pH d'une suspension d'acide libre de cefbupérazone dans l'eau à 3—6

b) ajouter de l'acétone à la solution

c) ajuster le pH à 1—3, et

d) isoler le produit.

8. Procédé pour la préparation des compositions selon la revendications 1 à 5, comprenant le mélange des composants a) et b) en une proportion suffisante pour produire une solution reconstituée ayant le pH voulu d'au moins 3,5 à 3,7.

**Revendications pour l'Etat contractant: AT**

1. Procédé de préparation d'une composition de mélange sec de cefbupérazone, stérile et stable, pour reconstitution avec un diluant stérile, pharmaceutiquement acceptable, pour produire une solution injectable, dont l'administration est peu douloureuse, constitué en ce que l'on mélange

a) une quantité thérapeutiquement active du dihydrate de l'acide libre de cefbupérazone, de l'acide libre de cefbupérazone anhydre, ou du hemi-acétonate de l'acide libre de cefbupérazone, et

b) de la lysine, de l'arginine, de l'acétate de lysine, de l'acétate d'arginine ou un de leurs mélanges, en une quantité suffisante pour produire une solution reconstituée ayant un pH de 4 à 7.

2. Procédé selon la revendication 1, dans laquelle la lysine, l'arginine, l'acétate de lysine, l'acétate d'arginine ou un de leurs mélanges, est présent en une quantité suffisante pour produire une solution reconstituée ayant un pH de 4 à 6.

3. Procédé de préparation d'une composition de mélange sec de cefbupérazone, stérile et stable, pour reconstitution avec un diluant stérile et pharmaceutiquement acceptable, pour produire une solution injectable, dont l'administration est peu douloureuse, constitué en ce que l'on mélange

a) une quantité thérapeutiquement active du dihydrate de l'acide libre de cefbupérazone, de l'acide libre de cefbupérazone anhydre, ou du hemi-acétonate de l'acide libre de cefbupérazone, et

b) de la lysine ou de l'arginine, en une quantité suffisante pour produire une solution reconstituée ayant un pH d'environ 4 à environ 5.

4. Procédé selon la revendication 1, dans lequel la lysine ou l'arginine sont présentes en une quantité suffisante pour produire une solution aqueuse reconstituée ayant un pH de 4 à 5.

5. Procédé selon la revendication 4, pour préparer une composition en doses individuelles contenant, lorsqu'elle est reconstituée, d'environ 300 à environ 350 mg d'activité de cefbupérazone par ml de solution reconstituée.

6. Procédé pour la preparation du hemi-acétonate de l'acide libre de cefbupérazone, comprenant les opérations consistant à:

a) ajuster le pH d'une suspension d'acide libre de cefbupérazone dans l'eau à 3—6

b) ajouter de l'acétone à la solution

c) ajuster le pH à 1—3, et

d) isoler le produit.

7. Procédé selon la revendications 1 à 5, comprenant le mélange des composants a) et b) en une proportion suffisante pour produire une solution reconstituée ayant le pH voulu d'au moins 3,5 à 3,7.

FIG. 1

INFRARED SPECTRUM OF CEFBUPERAZONE HEMIACETONATE